# EUROPÄISCHE PATENTSCHRIFT

(11) **EP 0 507 118 B1**
(45) Veröffentlichungstag und Bekanntmachung des Hinweises auf die Patenterteilung: **09.08.1995**
(21) Anmeldenummer: 92104131.5
(22) Anmeldetag: 11.03.1992
(51) Int. Cl.: C07C 209/36

(54) **Verfahren zur Herstellung von aromatischen Aminen**
Process for the preparation of aromatic amines
Procédé pour la préparation d'amines aromatiques

(30) Priorität: 30.03.1991 DE 4110457
(43) Veröffentlichungstag der Anmeldung: 07.10.1992
(73) Patentinhaber: BASF Aktiengesellschaft, 67063 Ludwigshafen (DE)
(72) Erfinder: Mross, Wolf Dieter, Dr., W-6710 Frankenthal (DE); Dockner, Toni, Dr., W-6701 Meckenheim (DE); Wolff, Dietrich, Dr., W-6831 Plankstadt (DE); Fischer, Karl, Dr., W-6521 Hohen-Suelzen (DE); Fastre, Gustaaf, B-2920 Kalmthout (BE)

(56) Entgegenhaltungen:
- DE-B- 1 114 820
- DE-B- 1 133 394
- W. GERHARTZ ET AL. 'ULLMANN'S ENCYCLOPEDIA OF INDUSTRIAL CHEMISTRY, Fifth,Completely revised Edition, Vol. A 5: Cancer Chemotherapy to Ceramic Colorants'1986 , VCH VERLAG , WEINHEIM, DE

## Beschreibung

Die vorliegende Erfindung betrifft ein neues und verbessertes Verfahren zur Herstellung von aromatischen Aminen durch katalytische Hydrierung von Nitroaromaten an Hydrierkatalysatoren bei erhöhten Temperaturen, wobei der Hydrierkatalysator durch ein Halogenid dotiert ist und/oder der Reaktionsmischung eine halogenhaltige organische Verbindung zugesetzt wird.

Aus Ullmanns Encyklopädie der technischen Chemie, 4. Auflage, Band 7, Seite 566 bis 576 (1979) ist die Hydrierung von Nitrobenzol in der Gasphase bekannt.

Ferner ist aus DE-A-11 14 820 bekannt, daß man Anilin durch katalytische Hydrierung von Nitrobenzol in Gegenwart von bestimmten Katalysatoren erhalten kann. Als Katalysatoren kommen die Schwermetalle der I. und V. bis VII. Gruppe des Periodensystems sowie der Eisen- und Platingruppe, z.B. Kupfer, Molybdän, Wolfram, Nickel, Kobalt oder Gemische dieser Elemente, sowie ihre Oxide, Sulfide oder Halogenide, gegebenenfalls zusammen mit Bor oder Borverbindungen, in Betracht. Sie können auch auf Träger, wie Tonerde, natürliche und künstliche Silikate, Bimsstein, Eisenoxid, Magnesia, Zinkoxid, Zirkonoxid, Titanoxid oder Thoriumoxid, aufgetragen sein. Die Träger können mit Brom, Iod, Fluor oder Chlor behandelt sein.

Diese Katalysatoren desaktivieren durch Verkoken, wobei der Druckverlust über das Katalysatorbett ansteigt und unumgesetztes Nitrobenzol den Reaktor verläßt. Durch Abbrennen des Koks mit Luft bei 250 bis 350°C und anschließender H₂-Behandlung können die Katalysatoren aber wieder reaktiviert werden.

Üblicherweise muß ein frischer Katalysator nach ca. 5 Tagen erstmals regeneriert werden. Auf dem Katalysator haben sich ca. 20 Gew.-% Koks abgeschieden. Die Regenerierungszeit beträgt ca. 60 h. Der Abstand zwischen zwei Regenerierungen verlängert sich bis zur 5. Regenerierung auf ca. 25 Tage. Die Regenerierungszeit bleibt jedoch konstant.

Ferner ist aus B. Dvorak et al., Deactivation of a supported copper catalyst in the industrial aniline plant, in B. Delmon, G.F. Froment, Catalyst Deactivation, 1987, Seiten 535 bis 544, Elsevier Science Publisher, Amsterdam 1987 bekannt, daß kleine Chlormengen den Katalysator desaktivieren.

Der vorliegenden Erfindung lag daher die Aufgabe zugrunde, den zuvor genannten Nachteilen abzuhelfen.

Demgemäß wurde ein neues und verbessertes Verfahren zur Herstellung von aromatischen Aminen durch katalytische Hydrierung von Nitroaromaten an Hydrierkatalysatoren bei 160 bis 450°C und Drücken von 1 bis 50 bar gefunden, welches dadurch gekennzeichnet ist, daß der Hydrierkatalysator 0,02 bis 1 Gew.-% Halogen und/oder die Reaktionsmischung 0,1 bis 50 ppm einer halogenhaltigen organischen Verbindungen bezogen auf den eingesetzten Nitroaromaten enthält.

Das erfindungsgemäße Verfahren läßt sich wie folgt durchführen:

Die Nitroaromaten und Wasserstoff werden bei Temperaturen von 160 bis 450°C, vorzugsweise 250 bis 330°C und Drücken von 1 bis 50 bar, vorzugsweise 1 bis 15 bar über einen Hydrierkatalysator diskontinuierlich bevorzugt kontinuierlich geführt.

Das Molverhältnis von Wasserstoff zum Nitroaromaten beträgt von 1:1 bis 500:1, bevorzugt 1,5:1 bis 50:1, besonders bevorzugt 2:1 bis 10:1.

Als Nitroaromaten eignen sich alle Nitroaromaten, die ein oder zwei Nitrogruppen tragen z.B. Phenyl-, Naphthyl- und Anthranylverbindungen.

Als Phenylverbindungen eignen sich beispielsweise Nitrobenzol oder ein- bis vierfach durch C₁- bis C₈-Alkyl, C₁- bis C₄-Alkoxy, Phenyl, Phenoxy, Hydroxy und/oder Halogen substituierte Nitrophenylverbindungen wie 2-Methyl-nitrobenzol, 3-Methyl-nitrobenzol, 4-Methyl-ntrobenzol, 4-Methyl-3-nitrophenyl, Nitrobenzol und 2-Methyl-nitrobenzol (Toluidin).

Als Hydrierkatalysatoren eignen sich die Metalle der I. und V bis VIII.-Nebengruppe des Periodensystems der Elemente, z.B. Kupfer, Molybdän, Wolfram, Nickel, Kobalt, die gegebenenfalls I. Haupt- und der II. Haupt- und Nebengruppe des Periodensystems der Elemente, wie Lithium, Natrium, Kalium, Rubidium, Cäsium, Magnesium, Calcium, Barium, Strontium, Zink und/oder Cadmium enthalten oder Gemische dieser Elemente, sowie ihre Oxide, Sulfide oder Halogenide, gegebenenfalls zusammen mit Chrom, Bor oder Borverbindungen. Sie können auch auf Träger, wie Kieselsäure, Silicalgel, Tonerde, natürliche und künstliche Silikate, Bimsstein, Eisenoxid, Magnesia, Zinkoxid, Zirkonoxid, Titanoxid oder Thoriumoxid, aufgetragen sein. Die Träger können mit Brom, Iod, Fluor oder Chlor behandelt sein.

Die Hydrierkatalysatoren enthalten als aktive Menge, bezogen auf das elementare Katalysatormetall vorzugsweise
5 bis 100 Gew.-% Kupfer, vorzugsweise 15 bis 30 Gew.-%
0,02 bis 1 Gew.-% Halogen,
0 bis 15 Gew.-% Chrom, vorzugsweise 0,05 bis 1 Gew.-%
0 bis 20 Gew.-% Zink, vorzugsweise 0,05 bis 1 Gew.-%
0 bis 30 Gew.-% Alkalimetall, vorzugsweise 0,05 bis 1,5 Gew.-%
0 bis 10 Gew.-% Erdalkalimetall, vorzugsweise 0,05 bis 2 Gew.-%,
mit der Maßgabe, daß die Summe der Gew.-% an Katalysatormetall 100 Gew.-% nicht übersteigt.

Der Einsatz von Halogen kann sowohl bei der Herstellung des Katalysators als auch beim Ausführen der Reaktion erfolgen.

Bei der Herstellung des Katalysators erfolgt die Halogenzugabe bevorzugt durch Zugabe von halogenidhaltigen Salzen zu der Imprägnierlösung.

Der Katalysator enthält 0,02 bis 1 Gew.-% Halogen. Bevorzugt werden 0,05 bis 0,5 Gew.-% Halogen. Als Halogen eignen sich Fluor, Chlor und Brom, bevorzugt Chlor und Brom, besonders bevorzugt Chlor in Form von Halogeniden der Elemente der Katalysatoren wie bevorzugt Kupfer(II)chlorid, Kalium-, Natrium-, Lithium-, Cäsium-, Magnesium-, Calcium-, Strontium-, Barium-, Zink-, Cadmiumchlorid, Chrom(III)chlorid, z.B. als Imprägnierlösung zugesetzt.

Die Halogenzugabe während der Reaktion erfolgt bevorzugt durch Zugabe einer halogenhaltigen, organischen Verbindung zu dem Einsatzstoff mit einem Halogengehalt von 0,1 bis 50 Gew.-ppm, bezogen auf den Feed an Nitroaromaten. Bevorzugt wird ein Halogengehalt von 0,2 bis 30 Gew.-ppm. Besonders bevorzugt werden chlorhaltige organische Verbindungen mit einem Chlorgehalt von 0,25 bis 20 Gew.-ppm, ganz besonders bevorzugt werden Chlornitrobenzol bzw. -toluol mit einem Chlorgehalt von 0,3 bis 10 Gew.-ppm, bezogen auf den Einsatzstoff Nitrobenzol bzw. Nitrotoluol.

Als Reaktoren eignen sich z.B. Rohrreaktoren und Wirbelbetten, bevorzugt sind Rohrreaktoren mit vertikalen Rohren und Wirbelbetten.

Die Reaktion kann in der Flüssigphase, bevorzugt in der Gasphase durchgeführt werden.

Überraschenderweise zeigte sich, daß sich durch gezielte Dotierung des Katalysators mit Halogen die Regenerierungsintervalle deutlich verlängern lassen.

So muß ein frischer, promotierter Katalysator erst nach ca. 35 Tagen abgebrannt werden. Der Koksgehalt vor der Regenerierung beträgt nur ca. 8 %. Die Regenerierung ist nach 24 h abgeschlossen.

Geeignete Nitroaromaten II bzw. aromatische Amine I sind z.B.

| Nitroaromaten (II) | aromatisches Amin (I) |
|---|---|
| Nitrobenzol | Anilin |
| 2-Nitrotoluol | 2-Methylanilin (Toluidin) |
| 3-Nitrotoluol | 3-Methylanilin |
| 4-Nitrotoluol | 4-Methylanilin |

Die nach dem erfindungsgemäßen Verfahren herstellbaren aromatischen Amine sind Zwischenprodukte.

### Beispiele

### Herstellung des Katalysators

### Katalysator A-1

In 1700 l Ammoniakwasser (25 Gew.-% NH₃) werden 430 kg Kupfercarbonat, 180 kg Hirschhornsalz, 13 kg Bariumnitrat, 28,4 kg Chromnitratlösung (6,4 Gew.-% Cr), 23,5 kg Zinknitratlösung (14,0 Gew.-% Zn), 4,1 kg Kupfer(II)chlorid und 24 kg Kalilauge gelöst. Die Lösung wird auf einen SiO₂-Grieß (Körnung: 0,1 bis 0,3 mm) aufgetränkt und der Katalysatorvorläufer unter laufendem Drehen der mit Dampf beheizten Imprägniertrommel getrocknet. Der Katalysator wird anschließend bei einer Verweilzeit von 1 h im Drehrohr bei 300°C kalziniert.

### Katalysator A-2

Die Herstellung erfolgt wie im Fall des Katalysators A-1, jedoch wurde der Zusatz des Kupfer(II)chlorids verdoppelt.

### Katalysator A-3

Die Herstellung erfolgte wie im Fall des Katalysators A-1, jedoch wurde der Zusatz des Kupfer(II)chlorids halbiert.

### Vergleichskatalysator AV

Die Herstellung erfolgte wie im Fall des Katalysators A, jedoch unterblieb der Zusatz des Kupfer(II)chlorids.

Die analytischen Daten der verwendeten Katalysatoren sind in der folgenden Tabelle zusammengefaßt.

**Tabelle**

| Katalysator | CuO | Cr₂O₃ | ZnO | BaO | K₂0 | Na₂0 | Cl |
|---|---|---|---|---|---|---|---|
| | | | [ Gew.-%] | | | | |
| A-1 | 27,5 | 0,27 | 0,39 | 0,67 | 0,86 | 0,22 | 0,29 |
| A-2 | 27,1 | 0,21 | 0,41 | 0,71 | 0,81 | 0,21 | 0,57 |
| A-3 | 27,6 | 0,26 | 0,44 | 0,66 | 0,91 | 0,18 | 0,13 |
| AV | 27,5 | 0,25 | 0,40 | 0,72 | 0,84 | 0,24 | - |

### Beispiel 1

In einem technischen Wirbelofen von 20 m³ Inhalt, ausgestattet mit einem Verdampfungskühlregister wurden 2600 kg Katalysator A1 und 5200 kg Katalysator B eingefüllt und durch Einleiten von 8000 Nm³/h Wasserstoff zum Wirbeln gebracht. Das Gas ist durch einen Vorerhitzer auf 200°C geheizt und erwärmt den Katalysator. Ab 200°C setzt eine Reduktionsreaktion ein, die die Temperatur im Wirbelbett auf 260°C steigert. Nach 1,5 h Reduktionszeit werden über in der Reaktorwand angebrachte Düsen 43600 kg flüssiges Nitrobenzol eingespritzt. Nitrobenzol wird verdampft und setzt sich zu Anilin und Wasser um. Die den Reaktor verlassenden Produktgase werden in einem nachgeschalteten Kühler kondensiert, in einem Behälter gesammelt und nach üblichen Methoden aufgearbeitet.

Die Reaktionstemperatur steigt auf 300°C, die Reaktionswärme wird über Wasserverdampfung im Kühlregister abgeführt. Nach 23 Tagen wird der Versuch ohne Einbuß von Umsatz, Selektivität und Wärmeabführung abgebrochen. Die Untersuchung einer Katalysatorprobe ergab 5 % Kohlenstoff.

### Beispiel 2 (Vergleichsbeispiel)

Der Versuch wurde wie unter 1 beschrieben durchgeführt mit dem Unterschied, daß anstelle von Katalysator A-1 Katalysator AV eingesetzt wurde.

Der Versuch mußte nach 8 Tagen abgebrochen werden, da sich im Wirbelbett starke Temperaturerhöhungen wegen unzureichender Wärmeabführung mit Durchbruch von unumgesetztem Nitrobenzol bemerkbar machten. Das Wirbelverhalten war durch starke Verkokung gestört. Die Untersuchung einer Probe ergab 15 % Kohlenstoff.

## Patentansprüche

1. Verfahren zur Herstellung von aromatischen Aminen durch katalytische Hydrierung von Nitroaromaten an Hydrierkatalysatoren bei 160 bis 450°C und Drücken von 1 bis 50 bar, dadurch gekennzeichnet, daß der Hydrierkatalysator 0,02 bis 1 Gew.-% Halogen und/oder die Reaktionsmischung 0,1 bis 50 ppm einer halogenhaltigen organischen Verbindung, bezogen auf den eingesetzten Nitroaromaten enthält.

2. Verfahren zur Herstellung von aromatischen Aminen nach Anspruch 1, dadurch gekennzeichnet, daß das Halogen ein Halogenid eines der Elemente des Katalysators ist.

3. Verfahren zur Herstellung von aromatischen Aminen nach Anspruch 1, dadurch gekennzeichnet, daß man CuCl₂, ein Chlorid eines Elements der I. Haupt-, der II. Haupt- und Nebengruppe oder CrCl₃ verwendet.

4. Verfahren zur Herstellung von aromatischen Aminen nach Anspruch 1, dadurch gekennzeichnet, daß man als halogenhaltige organische Verbindung einen entsprechenden halogenhaltigen Nitroaromaten verwendet.

5. Verfahren zur Herstellung von aromatischen Aminen nach Anspruch 1, dadurch gekennzeichnet, daß man Hydrierkatalysatoren einsetzt, die als aktive Masse 5 bis 100 Gew.-% Kupfer enthalten.

6. Verfahren zur Herstellung von aromatischen Aminen nach Anspruch 1, dadurch gekennzeichnet, daß man die Umsetzung bei Temperaturen von 250 bis 330°C durchführt.

7. Verfahren zur Herstellung von aromatischen Aminen nach Anspruch 1, dadurch gekennzeichnet, daß man die Umsetzung bei Drücken von 1 bis 15 bar durchführt.

8. Verfahren zur Herstellung von aromatischen Aminen nach Anspruch 1, dadurch gekennzeichnet, daß man als Nitroaromaten Nitrobenzol oder Nitrotoluol verwendet.

## Claims

1. A process for the preparation of aromatic amines by catalytic hydrogenation of nitroaromatic compounds on hydrogenation catalysts at from 160 to 450°C and pressures of from 1 to 50 bar, wherein the hydrogenation catalyst contains from 0.02 to 1% by weight of halogen and/or the reaction mixture contains from 0.1 to 50 ppm of a halogen-containing organic compound, based on the nitro aromatic compounds employed.

2. A process for the preparation of aromatic amines as claimed in claim 1, wherein the halogen is a halide of one of the elements of the catalyst.

3. A process for the preparation of aromatic amines as claimed in claim 1, wherein CuCl₂, a chloride of an element from main groups I or II or sub-groups II or CrCl₃ is used.

4. A process for the preparation of aromatic amines as claimed in claim 1, wherein the halogen-containing organic compound is a corresponding halogen-containing nitroaromatic compound.

5. A process for the preparation of aromatic amines as claimed in claim 1, wherein hydrogenation catalysts are employed which contain from 5 to 100% by weight of copper as active material.

6. A process for the preparation of aromatic amines as claimed in claim 1, wherein the reaction is carried out at from 250 to 330°C.

7. A process for the preparation of aromatic amines as claimed in claim 1, wherein the reaction is carried out at pressures of from 1 to 15 bar.

8. A process for the preparation of aromatic amines as claimed in claim 1, wherein the nitroaromatic compounds used are nitrobenzene or nitrotoluene.

## Revendications

1. Procédé de préparation d'amines aromatiques par hydrogénation catalytique de composés aromatiques nitrés en présence de catalyseurs d'hydrogénation à une température de 160 à 450°C et sous une pression de 1 à 50 bar, caractérisé en ce que le catalyseur d'hydrogénation contient de 0,02 à 1% en poids d'un halogène et/ou le mélange réactionnel contient de 0,1 à 50 ppm d'un composé organique halogéné, par rapport au composé aromatique nitré mis en réaction.

2. Procédé de préparation d'amines aromatiques selon la revendication 1, caractérisé en ce que l'halogène est un halogénure de l'un des éléments du catalyseur.

3. Procédé de préparation d'amines aromatiques selon la revendication 1, caractérisé en ce que l'on utilise CuCl₂, un chlorure d'un élément des groupes IA, IIA, IB et IIB de la classification périodique ou CrCl₃.

4. Procédé de préparation d'amines aromatiques selon la revendication 1, caractérisé en ce que l'on utilise, comme composé organique halogéné, un composé aromatique nitré halogéné correspondant.

5. Procédé de préparation d'amines aromatiques selon la revendication 1, caractérisé en ce que l'on utilise des catalyseurs d'hydrogénation qui contiennent de 5 à 100% en poids de cuivre en tant que masse active.

6. Procédé de préparation d'amines aromatiques selon la revendication 1, caractérisé en ce que l'on conduit la réaction à des températures de 250 à 330°C.

7. Procédé de préparation d'amines aromatiques selon la revendication 1, caractérisé en ce que l'on conduit la réaction sous des pressions de 1 à 15 bar.

8. Procédé de préparation d'amines aromatiques selon la revendication 1, caractérisé en ce que l'on utilise, comme composé aromatique nitré, du nitrobenzène ou du nitrotoluène.
